(19) Europäisches Patentamt
European Patent Office
Office européen des brevets

(11) Publication number: 0 132 391
A2

(12) # EUROPEAN PATENT APPLICATION

(21) Application number: 84304948.7

(22) Date of filing: 19.07.84

(51) Int. Cl.⁴: C 07 C 51/56, C 07 C 53/12

(30) Priority: 20.07.83 GB 8319619

(43) Date of publication of application: 30.01.85
Bulletin 85/5

(84) Designated Contracting States: BE DE FR GB IT NL

(71) Applicant: BP Chemicals Limited, Belgrave
House 76 Buckingham Palace Road, London, SW1W 0SU
(GB)

(72) Inventor: Cooper, Jeremy Bernard BP Chemicals
Limited, Belgrave House 76 Buckingham Palace Road,
London SW1W 0SU (GB)

(74) Representative: Crack, Richard David et al, c/o The
British Petroleum Company plc Patents Division
Chertsey Road, Sunbury-on-Thames Middlesex,
TW16 7LN (GB)

(54) **Process for the production of acetic anhydride.**

(57) Acetic anhydride and hydrogen iodide can be prepared by the reaction of methyl iodide, methanol or acetic acid, and carbon monoxide under carbonylation conditions using a catalyst comprising a Group VIII metal and a Lewis base promoter. By reacting the hydrogen iodide with methanol to produce methyl iodide and recycling the methyl iodide an integrated process for the production of acetic anhydride can be devised which uses only methanol or methanol and acetic acid as organic feedstocks. The process has the advantage over existing technology that methyl iodide and water produced by the reaction of hydrogen iodide and methanol can be separated without distillation.

EP 0 132 391 A2

# PROCESS FOR THE PRODUCTION OF ACETIC ANHYDRIDE

The present invention relates to a process for the production of acetic anhydride from carbon monoxide, acetic acid and methanol.

Acetic acid and acetic anhydride have been known as industrial chemicals for many years. Acetic anhydride constitutes the second largest end use for acetic acid and is widely employed in the production of cellulose acetate and other cellulose esters. Smaller quantities are used in the production of specialist esters, aspirin and pesticides. Acetic acid is used as a preservative and as an intermediate in the production of for example acetate esters.

Until recently acetic acid has almost exclusively been produced on an industrial scale by the oxidation of petroleum-derived fractions. Now, however, its production by the rhodium catalysed carbonylation of methanol is gaining increasing importance. On an industrial scale acetic anhydride is currently produced either by reaction of ketene and acetic acid, the ketene being obtained either by dehydration of acetic acid or by decomposition of acetone, or by oxidation of acetaldehyde which also yields acetic acid. Each of these conventional routes has well-known disadvantages which knowledge, together with the increasing attention being paid to chemical syntheses involving carbon monoxide and carbon monoxide/hydrogen mixtures, has led to investigations into the production of acetic anhydride utilising these materials.

In 1977 there was published the complete specification of British Patent No 1468940 (Halcon International Inc) which describes and claims a process for the preparation of an anhydride of a

monocarboxylic acid which comprises reacting a carboxylate ester satisfying the formula RCOOR or an ether satisfying the formula ROR with an acyl halide satisfying the formula RCOX, formed in situ or in a separate stage, under substantially anhydrous conditions, wherein X is iodide or bromide, the Rs may be the same or different and each R is a monovalent hydrocarbyl radical or a substituted monovalent hydrocarbon radical wherein the or each substituent is inert. The acyl halide may be produced by carbonylation of a halide satisfying the formula RX at superatmospheric pressure, R and X being as hereinbefore defined, and the carbonylation may be effected in the presence as catalyst of a Group VIII noble metal, ie indium, osmium, platinum, palladium, rhodium and ruthenium, and optionally a promoter selected from elements having atomic weights greater than 5 of Groups IA, IIA, IIIA, IVB, VIB, the non-noble metals of Group VIII and the metals of the lanthanide and actinide groups of the Periodic Table, of which suitable metals are lithium, magnesium, calcium, titanium, chromium, iron, nickel and aluminium. It is emphasised in GB 1468940 that it is important that the carbonylation reaction should be carried out under substantially anhydrous conditions, ie the reactants should be essentially dry.

In GB 1 523 346 (Hoechst Aktiengesellschaft) a process is disclosed for the preparation of acetic anhydride which comprises treating methyl acetate with carbon monoxide in the presence of a metal selected from ruthenium, rhodium, palladium, osmium, iridium and platinum, hereinafter referred to as a noble metal, in free or combined form and of a halogen in free or combined form. In this specification it is stated that the reaction is carried out in the absence of any noticeable quantities of water. However the starting material may also contain methanol, eg up to 25% or minor quantities eg not more than 5% of water based on the total weight of water + methyl acetate + methanol. In that case the process yields not only acetic anhydride, but also acetic acid. The acetic acid can be easily separated from the main product, acetic anhydride, eg by distillation.

In GB 2033385 A (Halcon Research and Development Corporation)

is described a process in which a substantially completely anhydrous methyl acetate is prepared from a wet methyl acetate containing significant amounts of water by bringing the wet methyl acetate into contact with acetic anhydride. It is stated that the process is of particular effectiveness and suitability for the dehydration of methyl acetate to be used as feed to a carbonylation reaction where a substantially anhydrous methyl acetate is desired, eg for the preparation of acetic anhydride as described in, inter alia, GB 1468940 referred to above. The specification also discloses in claim 3 a process of producing methyl acetate by the esterification of acetic acid with methanol in an esterification zone and carbonylating the resultant wet methyl acetate after dehydration, in a carbonylation zone to form acetic anhydride, and including the steps of reacting said wet methyl acetate with acetic anhydride in an amount at least substantially stoichiometric equivalent to the water present in said wet methyl acetate in a dehydration zone to produce substantially anhydrous methyl acetate and acetic acid, feeding said substantially anhydrous methyl acetate and said acetic acid to said carbonylation zone, recovering acetic anhydride and acetic acid from said carbonylation zone, separating said acetic anhydride and said acetic acid, directing a portion of said acetic anhydride to said dehydration zone, said portion being said amount at least substantially stoichiometrically equivalent to the water present in said methyl acetate fed to said dehydration zone, and feeding said acetic acid separated from said acetic anhydride to said esterification zone for reaction with methanol to produce said wet methyl acetate.

The methyl acetate used as feedstock for this process is produced by esterification of methanol by acetic acid in the presence of an acid catalyst.

$$CH_3OH + CH_3CO_2H \longrightarrow CH_3CO_2CH_3 + H_2O$$

Such an esterification produces equimolar amounts of methyl acetate and water. Thus if anhydrous methyl acetate or methyl acetate containing less than an equimolar amount of water is required it becomes necessary to remove some or all of the

co-produced water.

Methods by which the water may be removed from such methyl acetate/water mixtures have been described in our European patent applications 0087869 and 0087870. These methods along with others in general involve modified distillation techniques since

1) the water and methyl acetate are miscible and form a single phase, and

2) the methyl acetate forms a constant boiling point mixture with the water (an azeotrope) which makes removal of the azeotroped water difficult.

In any event the separation of water and methyl acetate by distillation is energy intensive and hence expensive to operate.

A process has now been invented which avoids the necessity to prepare directly acetic anhydride from a methyl acetate feedstock and hence dispenses with the need to separate methyl acetate and water in large quantities after an esterification step. Furthermore, since the feedstock used is a mixture of acetic acid and a $C_1$ compound derived from methanol the process also takes advantage of the favourable low prices of methanol.

Accordingly, the present invention provides a process for the production of acetic anhydride from methyl iodide, acetic acid and carbon monoxide characterised by reacting the methyl iodide and acetic acid with carbon monoxide under carbonylation conditions, in the presence of an effective amount of a carbonylation catalyst.

The products of the reaction described herein are, according to the following equation,

$$CH_3I + CH_3CO_2H + CO \longrightarrow (CH_3CO)_2O + HI$$

acetic anhydride and hydrogen iodide. The two products are readily separated e.g. by distillation, and both may be recovered pure. It is a particularly important feature of this invention that the hydrogen iodide is recoverable since it is able to react quantitatively with methanol to form methyl iodide and water

$$CH_3OH + HI \longrightarrow CH_3I + H_2O$$

The hydrogen iodide may therefore be used to produce fresh methyl iodide which may be, in turn, carbonylated along with more

acetic acid. Since during the reaction all the iodide is recycled within the process, the overall reaction is

$$CH_3OH + CO + CH_3CO_2H \rightarrow (CH_3CO)_2O + H_2O$$

The reaction between methanol and hydrogen iodide

$$CH_3OH + HI \rightarrow CH_3I + H_2O$$

is in some respects similar to the esterification reaction used in the prior art to product methyl acetate

$$CH_3OH + CH_3CO_2H \rightarrow CH_3CO_2CH_3 + H_2O$$

since both reactions require water removal from either methyl iodide or methyl acetate before carbonylation can occur. However, unlike the methyl acetate and water mixture which forms a single phase separable only by distillation, methyl iodide and water mixtures from two separate phases, easily separable by low energy methods such as decanting. Furthermore, although small amounts of methyl iodide can dissolve in water no azeotrope is formed and therefore separation by distillation is easy.

Accordingly an embodiment of the present invention provides a process for the production of acetic anhydride from methanol, acetic acid and carbon monoxide in a series of process steps characterised in that

1) in a carbonylation stage a mixture of methyl iodide and acetic acid is reacted, under carbonylation conditions, with carbon monoxide in the presence of the catalyst described previously to form acetic anhydride and hydrogen iodide
2) in a separation stage the hydrogen iodide and acetic anhydride are separated and
3) in an iodination stage the hydrogen iodide and methanol are reacted to produce methyl iodide and water, the methyl iodide being subsequently separated from the water and recycled to stage (1).

The product stream withdrawn from stage (1) can consist exclusively of acetic anhydride and hydrogen iodide but more generally consists of a mixture of acetic anhydride, hydrogen iodide and unreacted reactants. In this case, stage (2) can consist of several steps which allow (a) the unreacted reactants to be isolated

and recycled to stage (1); (b) the hydrogen iodide to be isolated and fed to stage (3) and (c) the acetic anhydride to be recovered for sale or secondary uses.

A typical process scheme for the operation of the embodiment is shown in the figure and operates as follows. Carbon monoxide (via line 4) and acetic acid (via line 12) are fed to the carbonylation reactor 2 along with recycled methyl iodide and acetic acid (via line 8) where reaction occurs. In the first part of the separation stage, which is conveniently integral with the carbonylation reactor 2 two streams are removed. The higher boiling stream 16, consisting of acetic anhydride and some acetic acid, is fed to a distillation column 18 where the residual acid is removed overhead. The lower boiling stream 14, consisting of carbon monoxide, hydrogen iodide, methyl iodide and residual acetic acid is fed to a scrubber 10. In the scrubber the carbon monoxide is removed overhead and returned to the carbonylation reactor (via line 6) and the methyl iodide and resiudal acetic acid removed at the bottom and returned to the reactor 2 via line 8. The hydrogen iodide is removed at an intermediate point in the scrubber and fed to an iodination unit 22 in which it is reacted with methanol (via line 24) to produce methyl iodide and water. The methyl iodide and water may be conveniently separated by decanting in unit 22 and the methyl iodide returned to the scrubber. The water, which may contain small amounts of unreacted methanol and trace amounts of water may be fed to astripper if desired to remove the methyl iodide and methanol as overheads.

Acetic acid which is used as feedstock can be that produced by any of the well known industrial processes or it can be produced by hydrolysis of part of the acetic anhydride product. In a further embodiment of the invention described however the acetic acid is generated in situ in the carbonylation stage from methanol and carbon monoxide using the carbonylation catalyst. Thus in a further embodiment of the invention a process is provided for the production of acetic anhydride from methyl iodide, methanol and carbon monoxide characterised by reacting the methyl iodide and acetic acid with

carbon monoxide under carbonylation conditions in the presence of an effective amount of a carbonylation catalyst.

Furthermore on replacing acetic acid by methanol as feed to the carbonylation reactor 2 in the figure a process is obtained for producing acetic anhydride from methanol and carbon monoxide using the same series of integrated process steps as described earlier.

In addition to the extremes of pure methanol and pure acetic acid described above, it will be obvious to the skilled man that methanol/acetic acid mixtures of any ratio can also be used as feedstocks.

The carbonylation catalyst conveniently contains a Group VIII metal and the Group VIII metal carbonylation catalyst is preferably rhodium. It can be supplied in almost any chemical form such as the metal, a simple inorganic salt, for example the halide or nitrate, a simple organic salt, e.g. the acetate or an organometallic complex or carbonyl complex. The catalyst concentration can be from 100 to 5000 ppm and can conveniently be from 400 to 1200 ppm based on the total weight of reactants.

A reaction promoter, in the form of a Lewis base, can also be added. Suitable Lewis bases include amines, phosphines and arsines which are capable of undergoing a quaternisation reaction with methyl iodide to form a quaternary ammonium salt which is soluble in the reaction mixture. Suitably the Lewis base is selected from nitrogen heterocycles, e.g. 2-picoline, pyridine, quinoline pyrrole, pyrrolidine, imidazole and 2-methyl imidazole. Imidazole for example is readily alkylated under the reaction conditions to N-methyl imidazole which readily forms soluble quaternary salts. The Lewis base may suitably added in an amount up to 20 mole % of the methyl iodide reactant preferably 2 to 10 mole %.

The carbonylation reaction is preferably effected in the liquid phase. In the liquid phase the reaction is conveniently carried out at elevated temperatures up to 250°C. Preferably the temperature is in the range 140 to 200°C.

Conveniently the process is carried out under a superatmospheric pressure of carbon monoxide preferably at least

25 bar and for example between 25 and 100 bar. The carbon monoxide used should be substantially pure although up to 10% by volume can be used.

As regards the ratio of methyl iodide to methanol or acetic acid in the carbonylation reaction it is preferred to use a molar ratio in the range 5:1 to 1:5. However, it will be obvious to the skilled man that in integrated processes of the type described herein it is important that the molar ratio of reactants corresponds substantially to that required by the expected stoichiometry of the carbonylation stage in order to reduce the levels of by products. Thus for the integrated processes described herein the molar ratio of methyl iodide to acetic acid or methyl iodide to methanol in the carbonylation stage should be substantially 1:1. Likewise in the iodination stage it is preferred to use a molar ratio of methanol to hydrogen iodide of 1:1.

The invention is now illustrated by the following Examples.

Example 1

A 100 ml Hastelloy 'B2' autoclave was charged with 39.1 g methyl iodide, 30.0 g acetic acid, 5.0 g 1-methylimidazole and 0.200 g rhodium acetate dimer. The vessel was pressurized with carbon monoxide to 65 bar and heated to 180°C during 3 hours with stirring. After cooling, (gas chromatography) g.c. analysis of the reaction mixture showed it to contain 4.5 g of acetic anhydride, hydrogen iodide and 35.1 g of acetic acid.

Example 2

A 100 ml Hastelloy 'B2' autoclave was charged with 39.5 g methyl iodide, 30.1 g acetic acid, 10.2 g 1-methylimidazole and 0.200 g of rhodium acetate dimer. The vessel was pressurized with carbon monoxide to 65 bar and heated at 150°C during 3 hours. After cooling, the reaction mixture was analysed by g.c. and shown to contain 4.1 g of acetic anhydride, hydrogen iodide and 32.7 g of acetic acid.

Example 3

A 100 ml Hastelloy 'B2' autoclave was charged with 38.6 g of methyl iodide, 30.2 g acetic acid, 5.0 g triphenylphosphine and

0.200 g rhodium acetate dimer and pressurized with carbon monoxide to 65 bar. The vessel was then heated at 150°C during 3 hours with stirring. After cooling, g.c. analysis of the reaction mixture showed it to contain 3.1 g of acetic anhydride, hydrogen iodide and 31.2 g of acetic acid.

Claims

1. A process for the production of acetic anhydride from (a) methyl iodide, (b) acetic acid and/or methanol (c) and carbon monoxide characterised by reacting the methyl iodide, acetic acid and/or methanol with carbon monoxide under carbonylation conditions in the presence of an effective amount of a carbonylation catalyst.

2. A process as claimed in Claim 1 characterised in that the carbonylation catalyst comprises

(1) a Group VIII noble metal preferably rhodium

(2) a Lewis base promoter.

3. A process for the production of acetic anhydride from methanol, acetic acid and carbon monoxide in a series of process steps characterised in that;

1) in a carbonylation stage a mixture of (a) methyl iodide and (b) acetic acid and/or methanol is reacted under carbonylation conditions with carbon monoxide in the presence of the carbonylation catalyst claimed in Claim 1 to form acetic anhydride and hydrogen iodide,

2) in a separation stage the hydrogen iodide and acetic anhydride are separated, and

3) in an iodination stage the hydrogen iodide and methanol are reacted to produce methyl iodide and water, the methyl iodide being subsequently separated from the water and recycled to stage (1).

4. A process as claimed in Claim 3 characterised in that in the iodination stage the methyl iodide and water are allowed to separate into two layers and are withdrawn separately.

5. A process as claimed in Claim 1 characterised in that the reaction is carried out at a temperature in the range 140 to 200°C and at a pressure in the range 25 to 100 bar.

6. A process as claimed in Claim 1 characterised in that the carbon monoxide contains up to 10% by volume hydrogen gas.

7. A process as claimed in Claim 2 characterised in that the Lewis base promoter is a nitrogen heterocycle.

8. A process as claimed in Claim 7 characterised in that the nitrogen heterocycle is imidazole or 2-methyl imidazole.

0132391



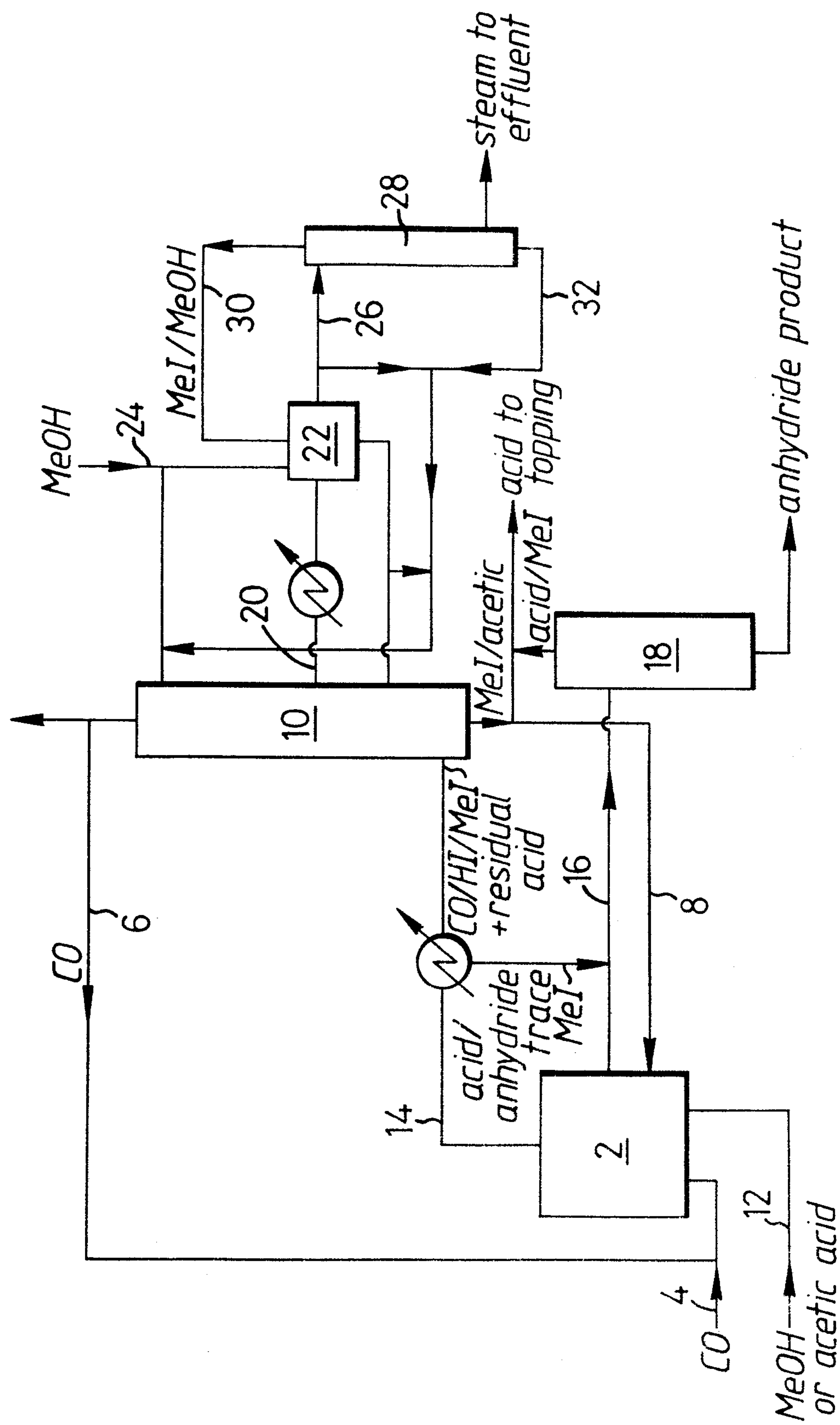

steam to effluent
28
MeI/MeOH
30
26
32
MeOH
24
22
acid to topping
anhydride product
20
MeI/acetic acid/MeI
18
10
CO/HI/MeI +residual acid
16
8
CO
6
acid/ anhydride trace MeI
14
2
12
CO
4
MeOH or acetic acid